(19) Europäisches Patentamt
European Patent Office
Office européen des brevets

(11) Publication number: **0 497 026 A1**

(12) # EUROPEAN PATENT APPLICATION

(21) Application number: **91300844.7**

(22) Date of filing: **01.02.91**

(51) Int. Cl.5: **A61L 17/00**, C08J 7/04, C08K 5/05, C08K 5/10

(43) Date of publication of application:
**05.08.92 Bulletin 92/32**

(84) Designated Contracting States:
**AT BE CH DE DK ES FR GB GR IT LI LU NL SE**

(71) Applicant: **UNITED STATES SURGICAL CORPORATION**
**150 Glover Avenue**
**Norwalk, Connecticut 06856(US)**

(72) Inventor: **Hermes, Matthew E.**
**288 Westport Road**
**Easton, Connecticut 06612(US)**
Inventor: **Kaplan, Donald S.**
**7 White Oak Lane**
**Weston, Connecticut 06833(US)**
Inventor: **Muth, Ross R.**
**97 Clearview Drive**
**RD2, Brookfield, Connecticut 06804(US)**

(74) Representative: **Marsh, Roy David et al**
**Hoffmann Eitle & Partner Patent- und Rechtsanwälte Arabellastrasse 4 Postfach 81 04 20**
**W-8000 München 81(DE)**

(54) **Method for improving the storage stability of a polymeric article susceptible to hydrolytic degradation and resulting article.**

(57) The storage stability of a polymeric article susceptible to hydrolytic degradation, e.g., an absorbable suture manufactured from a polymer of glycolic acid, glycolide, lactic acid, lactide or combination thereof, is improved by applying a storage stabilizing amount of at least one water soluble hygroscopic polyhydroxy compound and/or ester thereof, e.g., glycerol, monoacetin, diacetin, and the like, to the article as storage stabilizing agent, said agent being retained by the article prior to sealing of the enclosure in which the suture is packaged.

EP 0 497 026 A1

This invention provides a method for improving the storage stability of polymeric articles having an inherent tendency to undergo degradation when exposed to water or a humid atmosphere, probably as a result of hydrolysis. More particularly, the invention is directed to improving the storage stability of articles and devices such as absorbable surgical sutures, clips, staples, implants, prostheses and the like, fabricated from polymers which are susceptible to hydrolytic degradation, notably, polymers and copolymers of glycolic acid (i.e., hydroxyacetic acid), the cyclic dimer of glycolic acid ("glycolide"), lactic acid, the cyclic dimer of lactic acid ("lactide") and related monomers.

Polymers and copolymers of the foregoing kind and absorbable surgical devices made therefrom are well known. See, e.g., U.S. Patent Nos. 2,668,162; 2,703,316; 2,758,987; 3,225,766; 3,297,033; 3,422,181; 3,531,561; 3,565,869; 3,620,218; 3,626,948; 3,636,956; 3,736,646; 3,772,420; 3,773,919; 3,792,010; 3,797,499; 3,839,297; 3,867,190; 3,878,284; 3,982,543; 4,060,089; 4,137,921; 4,157,437; 4,234,775; 4,237,920; 4,300,565; and, 4,523,591; U.K. Patent No. 779,291; D. K. Gilding et al., "Biodegradable polymers for use in surgery -- polyglycolic/poly(lactic acid) homo-and co-polymers: 1, Polymer, Volume 20, pages 1459-1464 (1979), and D.F. Williams (ed.), Biocompatibility of Clinical Implant Materials, Vol. II, ch. 9: "Biodegradable Polymers" (1981). The biodegradability of these polymers/copolymers is believed to be due to the hydrolytic attack of their ester linkages by aqueous body fluids although the exact mechanism involved has been a matter of speculation.

An absorbable suture (or other aqueous body fluid-absorbable article) may experience prolonged storage before use, e.g., periods of several months and sometimes even several years. In order to prevent water or humidity in the storage environment from contacting the suture and compromising its in vivo strength to the point where the suture is no longer serviceable, it is common practice to package the suture in an essentially moisture impermeable enclosure. However as noted in U.S. Patent Nos. 3,728,839 and 4,135,622, any package material which prevents the entry of moisture will also prevent the escape of moisture. Thus, any moisture associated with or absorbed by the suture at the time it is packaged will tend to remain in the package for the entire period of its storage.

According to aforesaid U.S. Patent Nos. 3,728,839 and 4,135,622, the in-vivo strength of polyglycolic acid surgical elements such as sutures undergoes significant deterioration on long term standing in the package even on exposure of the contents to very small amounts of water for very short periods of time, e.g., 20 minutes or less, just prior to packaging due to the aforenoted tendency of a moisture impervious package to seal the moisture in with the suture.

To prevent hydrolytic degradation of the suture or to minimize its extent, U.S. Patent Nos. 3,728,839 and 4,135,622 disclose removing moisture from the suture before sealing the package so that no more than about 0.5 percent of water by weight of suture remains in the package once the package is sealed. This approach to improving the suture's storage stability, while effective, is in practice difficult and expensive to carry out. Prior to sealing the suture within its moisture impervious package, it is essential that the suture be "bone dry", a condition achieved by heating the suture for a sufficient period to remove the water therefrom, e.g., 180-188° for 1 hour under a 26 inch vacuum. However, once the water is removed, the suture cannot be allowed to contact a moisture-containing environment even for a limited duration since as previously noted, even brief exposure to moisture can cause severe deterioration of suture in vivo strength. It therefore becomes necessary following the water removal step to temporarily store the suture in a dry area, i.e., an environment which is essentially free of moisture, where the possibility of contact with moisture is largely eliminated.

Considered in their entirety, these operations for improving the storage stability of absorbable sutures and other surgical devices which are susceptible to hydrolytic degradation amount to a time consuming, expensive and relatively complex solution to the storage stability problem.

The present invention overcomes the aforenoted disadvantages associated with the storage stabilizing method described in U.S. Patent Nos. 3,728,839 and 4,135,622.

The invention provides a method for improving the storage stability of a polymeric article susceptible to hydrolysis, e.g., an absorbable surgical article such as a suture based in whole or in part on a polyester polymer or copolymer such as polyglycolic acid, lactide-glycolide copolymer, polydioxanone, polytrimethylene carbonate, their copolymers, etc., which requires neither a diminution of the article's pre-packaged moisture content nor temporary storage of the article in an artificially-maintained bone dry environment prior to completion of the packaging operation.

Thus, in accordance with this invention, a method is provided for improving the storage stability of a polymeric article susceptible to hydrolysis which comprises applying a storage stabilizing amount of at least one water soluble hygroscopic polyhydroxy compound or ester thereof to the polymeric article as storage stabilizing agent therefor.

Ordinarily, the foregoing method can be carried out upon the polymeric article without the need to

reduce its moisture level, either before or after applying the stabilizing agent thereto, to a very low level, e.g., to a state of being bone dry as in U.S. Patent Nos. 3,728,839 and 4,135,622, since entirely acceptable levels of storage stability can be achieved without resorting to such drastic moisture reduction efforts. Similarly, it is altogether unnecessary to maintain the article in a bone dry environment at any time following its manufacture and preceding the completion of its packaging as in the aforesaid patents. Once the article is contacted with the storage stabilizing agent which will thereafter be retained on and/or within the polymeric article, for example, by adhering to its surfaces and/or being sorbed by the polymeric composition of which the article is constructed, the article can be immediately packaged since all that is necessary to effect its long term hydrolytic stability will have been accomplished by the storage stabilizing agent application operation. Such being the case, the storage stabilizing method of the present invention possesses the advantages of simplicity, economy and a level of production efficiency unattainable by the storage stabilizing method described in U.S. Patent Nos. 3,728,839 and 4,135,622.

In addition to imparting an enhanced degree of storage stability upon polymeric articles which are subject to hydrolytic degradation, practice of the present invention may confer other benefits as well. So, for example, an absorbable suture which has been filled with a storage stabilizing amount of glycerol in accordance with the method herein has been found to exhibit better flexibility and "hand" characteristics than the untreated suture. Moreover, since the hygroscopic compounds useful in the practice of this invention are generally capable of dissolving a number of medico-surgically useful substances, they can be used as vehicles to deliver such substances to a wound or surgical site at the time the suture (or other absorbable surgical device) is introduced into the body.

The term "filled" as used herein refers to the association of the polymeric article with a storage stabilizing amount of storage stabilizing agent whether this association be one in which the storage stabilizing agent is absorbed by the polymeric article, is present on the surfaces thereof or is a combination of the two.

A characteristic which the polymeric articles to be contacted with a storage stabilizing agent in accordance with this invention share in common is their relatively high susceptibility to undergoing destructive hydrolysis over a period of storage. Generally, this is an inherent characteristic of polymers and copolymers possessing a significant number of short-chain ester linkages in their structure as, for example, is the case with polyglycolic acid, lactide-glycolide polymers, polydioxanone, polytrimethylene carbonate, their copolymers, and related materials. While the invention is particularly useful for application to absorbable sutures both of the monofilament and multifilament type (e.g., those of the braided variety which are especially hygroscopic) fabricated from polymers and copolymers of this kind, it is applicable to other types of surgically useful articles as well, e.g., those disclosed in U.S. Patent No. 4,135,622, including without limitation, absorbable surgical clips, staples, sponges, gauze, implants and prostheses for reconstructing bone tissue, blood vessels, and so forth.

The useful storage stabilizing agents are generally selected from the water soluble hygroscopic polyhydroxy compounds or esters of such compounds, preferably those having no appreciable toxicity for the body at the levels present. With these requirements in mind, those skilled in the art are readily capable of identifying any number of storage stabilizing agents useful in the practice of this invention. Among the specific stabilizing agents which can be used herein with generally good results are glycerol and its mono- and diesters derived from low molecular weight carboxylic acids, e.g., monoacetin and diacetin (respectively, glyceryl monoacetate and glyceryl diacetate), ethylene glycol, diethylene glycol, triethylene glycol, 1,3-propanediol, trimethylolethane, trimethylolpropane, pentaerythritol, sorbitol, and the like. Glycerol is especially preferred. Mixtures of storage stabilizing agents, e.g., sorbitol dissolved in glycerol, glycerol combined with monoacetin and/or diacetin, etc., are also useful.

To prevent or minimize run-off or separation of the storage stabilizing agent from the suture, a tendency to which relatively low viscosity compounds such as glycerol are somewhat prone, it can be advantageous to combine the agent with a thickener. Many kinds of pharmaceutically acceptable non-aqueous thickeners can be utilized including water-soluble polysaccharides, e.g., hydroxypropyl methylcellulose (HPMC), and the other materials of this type which are disclosed in European Patent Application 0 267 015 referred to above, polysaccharide gums such as guar, xanthan, and the like, gelatin, collagen, etc. An especially preferred class of thickeners are the saturated aliphatic hydroxycarboxylic acids of up to 6 carbon atoms and the alkali metal and alkaline earth metal salts and hydrates thereof. Within this preferred class of compounds are those of the general formula

$$R - \overset{\displaystyle OH}{\underset{\displaystyle H}{C}} - (CH_2)_nCOOR' \qquad \text{(Formula I)}$$

wherein R is hydrogen or a methyl group, R' is hydrogen or a metal selected from the group consisting of alkali metal and alkaline earth metal and n is 0 or 1, and hydrates thereof. Specific examples of such compounds include salts of lactic acid such as calcium lactate and potassium lactate, sodium lactate, salts of glycolic acid such as calcium glycolate, potassium glycolate and sodium glycolate, salts of 3-hydroxy propanoic acid such as the calcium, potassium and sodium salts thereof, salts of 3-hydroxybutanoic acid such as the calcium, potassium and sodium salts thereof, and the like. As stated hereinabove, hydrates of these compounds can also be used. Calcium lactate, especially calcium lactate pentahydrate, is a particularly preferred thickener.

Where a thickener is utilized, it will be incorporated in the filling composition in at least that amount required to increase the overall viscosity of the composition to the point where the composition no longer readily drains away from the suture in a relatively short period. In the case of a preferred storage stabilizing agent-thickener combination, namely, glycerol and calcium lactate, the weight ratio of glycerol to calcium lactate can vary from about 1:1 to about 10:1 and preferably is from about 6:1 to about 8:1.

If necessary or desirable, the stabilizing agent(s) can be dissolved in any suitable non-aqueous solvent or combination of solvents prior to use. To be suitable, the solvent must (1) be miscible with the storage stabilizing agent at the concentration of the latter, (2) have a sufficiently high vapor pressure to be readily removed by evaporation, (3) not appreciably affect the integrity of the polymeric article and (4) capable, in combination with the storage stabilizing agent, of wetting the surface of the surgical article. Applying these criteria to a preferred storage stabilizing agent, glycerol, lower alcohols such as methanol and ethanol are entirely suitable solvent carriers.

Application of the storage stabilizing agent to the polymeric article can be carried out in any number of ways. Thus, for example, the article can be submerged in the storage stabilizing agent or solution thereof until at least a storage stabilizing amount of agent is acquired or otherwise retained by the article, even after the optional removal of any excess agent and/or accompanying solvent (if present) such as by drainage, wiping, evaporation, etc. In many cases, contact times on the order of from just a few seconds, e.g., about 10 seconds or so, to several hours, e.g., about 2 hours and even longer, are sufficient to impart a substantial improvement in the storage stability of the treated article compared to the same type of article which has not been treated with a storage stabilizing agent.

The foregoing submersion method of contacting the polymeric article with storage stabilizing agent can be conducted continuously or in batch. Thus, in the case of an absorbable suture, a running length of the suture can be continuously passed through a quantity of the stabilizing agent at a velocity which has been previously determined to provide the necessary degree of exposure, or contact time, of the suture with the storage stabilizing agent. As the suture emerges from the storage stabilizing agent, it can be passed through a wiper or similar device to remove excess agent prior to the packaging operation. In a batch operation, a quantity of suture is merely submerged within the storage stabilizing agent for the requisite period of time with any excess agent being removed from the suture if desired.

Alternatively, the storage stabilizing agent and solutions thereof can be applied by spraying, brushing, wiping, etc., on the surfaces of the polymeric articles such that the latter receive and retain at least a storage stabilizing amount of the agent. Yet another procedure which can be used to apply the storage stabilizing agent involves inserting the polymeric article in a package containing an effective amount of the agent such that intimate contact between the polymeric article and the agent will be achieved.

Whatever the contacting procedure employed, it is necessary that the article being treated acquire a storage stabilizing amount of the storage stabilizing agent. In general amounts of from about 2 to about 25, and preferably from about 5 to about 15 weight percent, of storage stabilizing agent(s) (exclusive of any solvent) by weight of the polymeric article contacted therewith is sufficient to provide significantly improved storage stability compared to that of the untreated article.

The method of the invention can be practiced in conjunction with other known and conventional procedures such as sterilization. Known and conventional packaging techniques and materials are also contemplated. As previously stated, an advantage of the present invention lies in its ability to provide enhanced storage stability in a polymeric article susceptible to hydrolytic degradation without having to eliminate all but a small amount of moisture from the article and maintain the article in an especially dry environment until the final package sealing operation as disclosed in U.S. Patent Nos. 3,728,839 and

4,135,622. While the present invention can be practiced with a suture or other article which has been treated in this manner, there is no necessity of doing so and for reasons of simplicity, economy and production efficiency, it is preferred that the article to be contacted with storage stabilizing agent in accordance with this invention not receive the treatment described in the aforesaid patents.

It is preferred that the method of this invention be practiced upon a polymeric article whose moisture level has equilibrated to that of the surrounding atmosphere, e.g., from about 5 percent to about 40 percent relative humidity or even higher. Such a moisture content in the atmosphere will typically result in a stabilized surgical article possessing an amount of moisture in the range of from about 0.3 to about 1.0 weight percent or more. Moisture levels within this range, while not tolerated by the packaging method and packaged synthetic surgical element of U.S. Patent Nos. 3,728,839 and 4,135,622, have no appreciably deleterious effect on the long term in vivo strength of polymeric articles contacted with a storage stabilizing agent in accordance with the present invention. Thus, the polymeric article treated with storage stabilizing agent can, if desired, be packaged at relatively high levels of relative humidity, e.g., those just mentioned.

It can be advantageous to apply one or more coating compositions to the storage stabilized article of this invention where particular functional properties are desired. Thus, for example, in the case of an absorbable suture which has been treated with glycerol for improved long term storage, the storage stabilized article can be coated with a polyethylene oxide-polypropylene oxide block copolymer or polyalkylene glycol, either of which has been further polymerized with glycolide monomer and lactide monomer or glycolide/lactide copolymer to improve surface lubricity and facilitate knot tie-down. Suitable materials for these purposes include the compositions disclosed in U.S. Patent Nos. 3,867,190; 3,942,532; 4,047,533; 4,452,973; 4,624,256; 4,649,920; 4,716,203; and, 4,826,945.

As previously noted, it can be advantageous to employ the storage stabilizing agent as a carrier for one or more medico-surgically useful substances, e.g., those which accelerate or otherwise beneficially modify the healing process when applied to a wound or surgical site. In general, any biologically active material which is soluble in and otherwise compatible with the selected storage stabilizing agent can be incorporated therein in therapeutically useful amounts. So, for example, a suture can be filled with storage stabilizing agent containing a therapeutic agent which will be deposited at the sutured site. The therapeutic agent may be chosen for its antimicrobial properties, capability for promoting wound repair and/or tissue growth or for specific indications such as thrombolysis. Antimicrobial agents such as broad spectrum antibiotics (gentamycin sulphate, erythromycin or derivatized glycopeptides) which are slowly released into the tissue can be applied in this manner to aid in combating clinical and sub-clinical infections in a surgical or trauma wound site. To promote wound repair and/or tissue growth, one or several growth promoting factors can be added to the storage stabilizing agent, e.g., fibroblast growth factor, bone growth factor, epidermal growth factor, platelet derived growth factor, macrophage derived growth factor, alveolar derived growth factor, monocyte derived growth factor, magainin, and so forth. Some therapeutic indications are: glycerol with tissue or kidney plasminogen activator to cause thrombosis, superoxide dismutase to scavenge tissue damaging free radicals, tumor necrosis factor for cancer therapy or colony stimulating factor and interferon, interleukin-2 or other lymphokine to enhance the immune system.

Preparing the storage stabilizing agent of the present embodiment is a relatively simple procedure. For example, in the case of glycerol and calcium lactate, the desired amount of glycerol is first introduced to a container, followed by the addition thereto of the desired amount of calcium lactate. If no solvent is to be used, the mixture is then thoroughly mixed. In the event a solvent is desired, the solvent such as methanol is added to the mixture of glycerol and calcium lactate and the solution is then thoroughly mixed to dissolve the compounds.

Generally, the stabilizing agent of this embodiment is comprised of a mixture of a compound within formula I hereinabove, such as calcium lactate, and a water soluble hygroscopic polyhydroxy compound, such as glycerol, in a weight ratio of between about 1:1 to about 1:10, most preferably 1:7, respectively. When a solvent, such as methanol, is utilized in the preparation of the stabilizing agent, the solvent is employed in amounts to provide a solution concentration of from about 20% to about 50%, preferably about 30% to about 45%, by weight of the compound of formula I hereinabove, such as glycerol, based on the total weight of the solution.

The following examples are illustrative of the storage stabilizing method and storage stabilized polymeric article of this invention. Tables 1 and 2 illustrate the storage stabilizing effects of the hygroscopic polyhydroxy compound. Tables 3 and 4 illustrate the storage stabilizing effects of the mixture containing the hygroscopic polyhydroxy and the compound represented by Formula I.

EXAMPLES 1-5

In the following examples, five specimens of absorbable suture were prepared, packaged, sealed and maintained under accelerated storage conditions and tested for in vitro strength (U.S.P. Knot Pull Test) as follows:

| Example | Absorbable Suture | Moisture Content at Packaging (wt. %) | Percent Relative Humidity at Packaging | Glycerol Storage Stabilizing Agent (wt.%) |
|---|---|---|---|---|
| 1 (control) | VICRYL[1] | ≦0.05 | - | - |
| 2 | VICRYL cleaned[2] | 0.3 | 6 | 10 |
| 3 | VICRYL cleaned[2] | 1.0 | 40 | 10 |
| 4 (control) | VICRYL[3] | 0.22 | 6 | - |
| 5 (control) | VICRYL[3] | 0.38 | 40 | - |

[1] VICRYL (Ethicon, Inc.) is a braided absorbable suture believed to be fabricated from a copolymer derived from 90 mole percent glycolide and 10 mole percent lactide. As packaged, the suture is coated with a lubricant in order to improve knot tie-down properties. Size 1-0 was used in these evaluations.
[2] VICRYL suture from which the original coating has been substantially removed.
[3] VICRYL suture packages were opened then resealed after equilibrating at the relative humidity shown.

To simulate the effects of long term storage on in vivo strength, the packaged sutures were stored for the periods designated in the table below at 56°C (132°F) and 10% relative to humidity.

Simulating in vivo strength, the sutures were placed in 37°C (98.6°F) buffered saline. The results of the Knot Pull test were as follows:

Table 1: Results of In Vitro Strength Following Accelerated Storage

| | Example 1 | Example 2 | Example 3 | Example 4 | Example 5 |
|---|---|---|---|---|---|
| $T_o$ (no storage) | | | | | |
| $t_o$ | 4.9 kg | 5.6 | 5.4 | 4.8 | 5.4 |
| $t_1$ | 4.9 kg | 4.0 | 4.9 | 4.3 | 4.1 |
| $t_2$ | 3.1 kg | 3.5 | 3.6 | 2.3 | 2.3 |
| $t_3$ | 1.3 kg | 1.5 | 1.5 | - | - |
| $T_1$ (one week storage) | | | | | |
| $t_o$ | 4.8 kg | 5.2 | 5.0 | 4.3 | 4.3 |
| $t_1$ | 4.4 kg | 4.8 | 4.5 | 2.9 | 3.5 |
| $t_2$ | 3.1 kg | 3.6 | 2.7 | 1.9 | 1.6 |
| $t_3$ | 1.5 kg | 1.7 | 1.1 | - | - |
| $T_2$ (two week storage) | | | | | |
| $t_o$ | 5.3 kg | 5.2 | 3.7 | 4.5 | 3.6 |
| $t_1$ | 4.6 kg | 5.2 | 4.3 | 1.9 | 3.2 |
| $t_2$ | 2.9 kg | 2.2 | 3.0 | 1.4 | 0.6 |
| $t_3$ | 2.4 kg | 2.8 | 0.9 | - | - |
| $T_3$ (three week storage) | | | | | |
| $t_o$ | 5.5 kg | 6.0 | 3.8 | 4.7 | 3.3 |
| $t_1$ | 4.8 kg | 4.7 | 2.8 | 2.1 | 3.2 |
| $t_2$ | 2.2 kg | 2.6 | 0.6 | 2.0 | 1.0 |
| $t_3$ | 1.2 kg | 1.2 | 0.6 | - | - |
| $T_4$ (four week storage) | | | | | |
| $t_o$ | 4.8 kg | 4.8 | 3.8 | 4.4 | 2.8 |
| $t_1$ | 3.8 kg | 4.3 | 1.8 | 2.9 | 1.6 |
| $t_2$ | 2.5 kg | 2.9 | 1.2 | 1.5 | 0.7 |
| $t_3$ | 0.9 kg | 1.4 | 0.4 | - | - |
| $T_6$ (six week storage) | | | | | |
| $t_o$ | 4.9 kg | 4.7 | 3.9 | 4.4 | 2.4 |
| $t_1$ | 3.6 kg | 4.0 | 2.2 | 2.9 | 1.2 |
| $t_2$ | 2.4 kg | 1.9 | 1.2 | 1.3 | 0.5 |

As these data show, despite the moisture content being six times higher for the suture of Example 2 compared to the commercial control sample of Example 1, the measured in vitro strengths of the suture of Example 2 were about as good as, and in some cases exceeded, that of the suture of Example 1. Comparing the data of Example 2 with that of Example 4, it is seen that despite its higher water content, the glycerol-treated suture of this invention retained a significantly higher percentage of its initial in vitro strength compared to the non-treated suture. Even when the suture possesses a moisture content of 1.0 weight percent (Example 3), a level which is double the maximum 0.5 weight percent level of the suture storage stabilizing method and storage stabilized suture of U.S. Patent Nos. 3,728,839 and 4,135,622, it possessed much better storage stability than a suture of considerably lower moisture content (Example 5) even though both sutures were packaged in an environment of identical relative humidity.

## EXAMPLES 6-7

Specimens of 1-0 Vicryl braided absorbable suture (approximately 0.05 weight percent moisture as originally packaged) which had been stripped of their original coatings were filled with about 10 weight percent glycerol and then coated with a copolymer prepared from equal parts by weight of a polyethylene glycol (molecular weight of about 7500) and a 20/80 glycolide-lactide copolymer in order to enhance the suture's knot rundown and knot tie-down characteristics. The coated suture was then equilibrated at relative humidities of 15 and 20%, respectively, at ambient temperature (Examples 6 and 7). Finally, the suture

specimens were subjected to accelerated storage and tested for in vitro strength as in Examples 1-5. The results of the testing are set forth in Table 2 as follows:

Table 2: In Vitro Strength Following Accelerated Storage

| | Example 6 (0.45 wt.% moisture at packaging) | Example 7 (0.70 wt.% moisture at packaging) |
|---|---|---|
| $T_o$ (no storage) | | |
| $t_o$ | 5.32 | 5.42 |
| $t_1$ | 4.70 | 4.85 |
| $t_2$ | 3.00 | 3.18 |
| $T_2$ (two week storage) | | |
| $t_o$ | 5.16 | 4.98 |
| $t_1$ | 4.7 | 3.75 |
| $t_2$ | 2.7 | 2.3 |
| $T_3$ (three week storage) | | |
| $t_o$ | 4.7 | 4.45 |
| $t_1$ | 3.7 | 3.35 |
| $t_2$ | 1.8 | 1.3 |
| $T_4$ (four week storage) | | |
| $t_o$ | 4.69 | 3.76 |
| $t_1$ | 2.7 | 1.75 |
| $t_2$ | 2.1 | 1.7 |
| $T_6$ (six week storage) | | |
| $t_o$ | 3.8 | 3.1 |
| $t_1$ | 2.73 | 1.34 |
| $t_2$ | 1.62 | 1.06 |

These data further demonstrate the effectiveness of applying glycerol to an absorbable suture in accordance with the present invention to improve the storage stability of the latter.

## EXAMPLE 8

Glycerol filled and glycerol/calcium lactate filled braided sutures were centrifuged using a Clay Adams bench top lab centrifuge in order to compare retention as a percentage of baseline fill. Four samples were spun after collecting baseline data on the uncentrifuged sample. The centrifuge was run for 15 minutes at top speed, centrifugal force 3,000 Gs. The results are shown in Table 3.

Table 3

| Sample | Uncentrifuged wt% G | Uncentrifuged wt% CaL | Centrifuged wt% G | Centrifuged wt% CaL | % Retention G | % Retention CaL |
|---|---|---|---|---|---|---|
| A: Size 3/0 Synthetic Absorbable Suture | 21.7 | --- | 10.5 ± 3 | --- | 48 ± 14 | - |
| B: Size 1/0 Synthetic Absorbable Suture | 3.4 | 2.7 | 3.3 | 2.8 | about 100 | about 100 |
| C: Size 3/0 Synthetic Absorbable Suture | 14.9 | 2.2 | 12.9 | 1.7 | 87 | 78 |
| D: Size 3/0 Synthetic Absorbable Suture | 15.4 | 3.8 | 9.9 | 2.7 | 64 | 71 |

G = glycerol
CaL = calcium lactate • 5 $H_2O$
absorbable sutures = fibers from glycolide/lactide copolymers

The foregoing data indicate that adding calcium lactate to glycerol gives an increase in glycerol retention.

EXAMPLE 9

Samples of calcium lactate/glycerin - filled braided sutures show equally improved stability to storage compared to glycerin filled braid without calcium lactate as shown in Table 4 (Compare C and D to A and E). In both cases, the stability is excellent compared to braid without glycerin and equilibrated at about the same moisture level.

Table 4

| Sample | C | D | A | E | F |
|---|---|---|---|---|---|
| % Glycerin | 14.9 | 15.4 | 21.7 | 10 | -0- |
| % Ca lactate | 2.2 | 3.8 | -0- | -0- | -0- |
| % Water | 0.55 | 0.55 | 0.45 | 0.45 | 0.45 |
| Storage Time in weeks at 56°C | % Strength Retained After 2 Weeks In Vitro at 37°C And After Accelerated Storage at 56°C | | | | |
| 0 | 50 | 50 | 50 | 50 | 50 |
| 1 | 64 | 55 | 53 | 54 | 35 |
| 2 | 53 | 56 | 50 | 39 | 13 |
| 3 | 49 | 50 | 45 | 32 | 22 |
| 4 | 65 | 56 | 39 | 36 | 15 |
| 5 | | | 36 | 43 | |
| 6 | | | | 36 | 11 |

## Claims

1. A method for improving the storage stability of a polymeric article susceptible to hydrolysis which comprises applying a storage stabilizing amount of at least one water soluble hygroscopic polyhydroxy compound and/or ester thereof to the polymeric article as storage stabilizing agent therefor.

2. The method of Claim 1 wherein the polymeric article is an absorbable suture fabricated in whole or in part from a polymer or copolymer or glycolic acid, glycolide, lactic acid, lactide, dioxanone, trimethylene carbonate, caprolactone, polyalkylene glycol, or combination thereof.

3. The method of Claim 1 or 2 wherein the storage stabilizing agent further comprises at least one thickener.

4. The method of Claim 3 wherein the thickener is a compound having the general formula

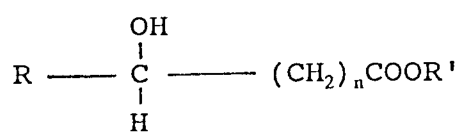

wherein R is hydrogen or a methyl group, R' is hydrogen or a metal selected from the group consisting of alkali metal and alkaline earth metals and n is 0 or 1, and hydrates thereof.

5. The method of Claim 1, 2, 3 or 4 wherein the storage stabilizing agent comprises a mixture of glycerol

and calcium lactate.

6. A polymeric article which is susceptible to hydrolysis possessing a storage stabilizing amount of at least one water soluble hygroscopic polyhydroxy compound or ester thereof as storage stabilizing agent.

7. The polymeric article of Claim 6 which is an absorbable suture fabricated in whole or in part from a polymer or copolymer of glycolic acid, glycolide, lactic acid, lactide, dioxanone, trimethylene carbonate, caprolactone, polyalkylene glycol, or combination thereof.

8. A polymeric article of Claim 6 or 7 wherein the storage stabilizing agent further comprises at least one thickener.

9. The polymeric article of Claim wherein the thickener is a compound having the general formula

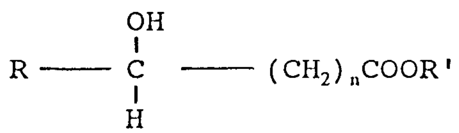

wherein R is hydrogen or methyl, R' is alkali metal or alkaline earth metal and n is 0 or 1, and hydrates thereof.

10. The polymeric article of Claim 6, 7, 8 or 9 wherein the storage stabilizing agent comprises a mixture of glycerol and calcium lactate.

European Patent Office

# EUROPEAN SEARCH REPORT

Application Number

EP 91 30 0844

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 376 656 (PFIZER HOSPITAL PRODUCTS GROUP)<br>* page 2, line 17 - line 34 *<br>* page 3, line 6 - line 11; claims * | 1,2,6,7 | A61L17/00<br>C08J7/04<br>C08K5/05<br>C08K5/10 |
| X | DE-A-3 635 679 (DYNAMIT NOBEL AG)<br>* page 2, line 30 - line 41; claims * | 1,2,6,7 | |
| D,X | US-A-3 531 561 (YVES M. TREHU)<br>* column 2, line 13 - line 23; claims; examples * | 1,2,6,7 | |
| A | WO-A-8 400 303 (RIJKSUNIVERSITEIT TE GRONINGEN)<br>* page 2, line 16 - line 22; claims * | 1,2,6,7 | |
| A | CHEMICAL ABSTRACTS, vol. 83, no. 10, 8 September 1975, Columbus, Ohio, US; abstract no. 80549N, page 70 ;<br>& SU-A-454 236 (INST. PHYS.-TECH. PROB. OF EN., AC. OF SCIENCES, LITHUANIAN S.S.R.) 25 December 1974 | 1 | TECHNICAL FIELDS SEARCHED (Int. Cl.5) |
| D,A | EP-A-0 267 015 (ETHICON INC.)<br>* claims * | 1 | A61L<br>C08K<br>C08J |

The present search report has been drawn up for all claims

| Place of search | Date of completion of the search | Examiner |
|---|---|---|
| THE HAGUE | 09 DECEMBER 1991 | Los Arcos E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)